# EUROPEAN PATENT SPECIFICATION

(11) **EP 4 461 257 B1**
(45) Date of publication and mention of the grant of the patent: **29.04.2026**
(21) Application number: 24180323.8
(22) Date of filing: 05.06.2024
(51) Int. Cl.: A61C 8/02, A61C 8/00, A61F 2/28

(54) **FREE GINGIVAL GRAFT KIT**
FREIER ZAHNFLEISCHTRANSPLANTATSATZ
KIT DE GREFFE GINGIVALE LIBRE

(30) Priority: 06.04.2023 KR 20230045191
(43) Date of publication of application: 13.11.2024
(73) Proprietor: Ou, Yi-Kun, Taoyuan City 330061 (TW)
(72) Inventor: Ou, Yi-Kun, Taoyuan City 330061 (TW)
(74) Representative: Patentanwälte Hemmer Lindfeld Frese Partnerschaft mbB

(56) References cited:
- WO-A1-2011/038354
- US-A1- 2010 217 331
- US-A1- 2012 077 140

## Description

### BACKGROUND

The inventive concept relates to auxiliary tools for use in a free gingival graft, and more particularly, to a free gingival graft kit which may reduce difficulties in a free gingival graft to improve surgical and medical treatment quality.

Generally, before treating patient's teeth, dentists primarily inspect a patient's current gingival state. When a patient has a problem of gingival recession, gingival restoration surgery is needed to improve the patient's gingival state. There are two types of gingival restoration surgeries, that is, connective tissue transplant surgery and free gingival tissue graft surgery, and one of which needs to be selected depending on different conditions.

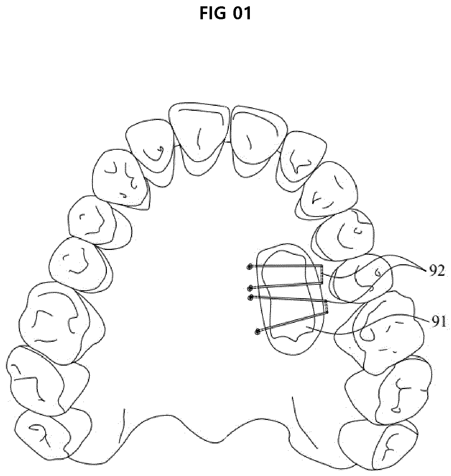

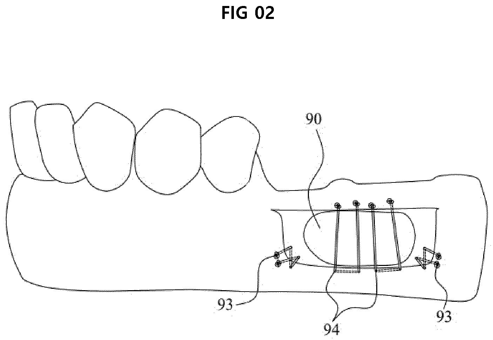

Referring to FIG. 1, in existing free gingival tissue graft surgery, pieces of a gingiva-containing graft 90 are collected directly from the upper inner side of patient's oral cavity. Next, a treatment medication 91, such as a compression hemostasis gauze or a growth factor, is arranged in a wound formed after the graft is collected, and the wound is sutured with sutures 92 so that the treatment medication 91 is fixed to the wound. As illustrated in FIG. 2, a position within the patient's oral cavity, with which the problem of gingival recession lies, is incised, and mucous pieces are pulled from the incision area and fixed to patient's periosteum by using first sutures 93. The collected gingiva-containing graft 90 is arranged in a gingival wound corresponding to an area where the mucous is incised and pulled, and thus, the entire piece of the gingiva-containing graft 90 is brought to contact with the patient's gingival wound. Lastly, a plurality of second sutures 94 are sequentially extended through the patient's gingival, periosteum, and gingival, and thus, the second sutures 94 are combined with the outer side of the gingiva-containing graft 90 to be in close contact with the gingival wound.

In the existing free gingival graft surgery, the incised mucous and the collected gingiva-containing graft are fixed in place by using sutures, and the sutures are stitched to periosteum having a very thin thickness. Accordingly, a surgeon requires more time to stitch the sutures with care. For surgeons with little experience, thin periosteum tends to be inappropriately pulled by the sutures so as to be damaged. Under some bad conditions, as the incised mucous is not fixed in place well by the sutures, it leads to a failure of free gingival graft surgery.

In addition, the treatment medication 91 arranged in the wound formed by collecting the gingiva-containing graft 90 is maintained in place only by the sutures. As the sutures are not able to cover the entire wound, when foreign materials contact the wound, patients frequently fee pain; meanwhile, a partially exposed wound tends to bleed. As the existing free gingival graft surgery has many difficulties and demerits for performing, there is a need to find a method that can minimize the difficulties in the performing of free gingival graft surgery and may help to reduce an unnecessary time for surgery. Accordingly, the present inventor proposes a free gingival graft kit by which a dentist may conveniently perform free gingival graft surgery and guarantee improved surgery effects.
US 2012/077140 A1 discloses a protective covering cap fixed with Temporal Anchorage Devices over the gingiva of a patient.

### SUMMARY

The inventive concept provides a graft surgery kit as defined in claim 1 used for free gingival graft surgery, which includes a non-scratching temporal anchorage device(non-scratching TAD) for supporting mucous incised at a certain distance from a wound within a patient's oral cavity to be treated so that the mucous does not repel the wound and there is no need to be stitched to very thin periosteum in the oral cavity as in existing gingival graft surgery. A graft surgery time may be greatly reduced, and surgical and subsequent medical treatment quality may be improved much.

The inventive concept provides a free gingival graft kit including a protective covering member for covering a local wound within a patient's oral cavity from which a piece of graft is donated, thereby promoting compression hemostasis of the local wound, preventing treatment medication from falling from the local wound, and preventing the local wound from touching the patient's tongue or food after surgery. Accordingly, the complicated procedure to stitch graft and treatment medication to the local wound may be simplified so that a time for hemostasis of a wound may be reduced.

The inventive concept provides a free gingival graft kit including a non-scratching TAD having no sharp corner to prevent the patient's oral cavity from being damaged during surgery. The non-scratching TAD also allows winding of a suture therearound, and thus, there is no need to stitch a suture to patient's periosteum so as to be inappropriately pulled. Accordingly, the difficulties of stitching a suture during free gingival graft surgery are greatly reduced so that a suture stitching time may be reduced.

The inventive concept provides a free gingival graft kit including a protective covering member that can be selected according to an actual treatment condition or at patient's discretion. The protective covering member used for graft surgery may be formed by 3D printing using a solid material or a medical-grade silicon material that is deformable.

The inventive concept provides a free gingival graft kit including an elastic covering film to be inserted above a non-scratching TAD, which may prevent difficulty in locating the non-scratching TAD after a local wound heals because regenerated gingiva does not cover the entire non-scratching TAD.

The inventive concept provides a free gingival graft kit including a locating needle to be used with a medical-grade silicon protective covering member. The locating needle forms a plurality of locating points on a silicon sheet so that the silicon sheet may be prevented from being moved above a local wound within a patient's oral cavity and also may facilitate passing of TADs through a patient's palate where graft is collected.

According to the inventive concept, a free gingiva graft kit includes a non-scratching TAD and a wound covering assembly. The non-scratching TAD includes a drilling section having a pointed tip and an expanded end portion, wherein a conical surface is defined between the pointed tip and the expanded end portion, a male thread provided on a circumference of the conical surface of the drilling section, a neck section connected to the expanded end portion of the drilling section and including a shank having a smooth external surface, and a non-scratching section including an upper semi-spherical portion and a lower semi-spherical portion, wherein the upper semi-spherical portion includes an engaging end portion to be engaged with a dental handpiece, the engaging end portion being provided with an opening that does not include a sharp corner, the lower semi-spherical portion includes a stop end portion, the stop end portion having a diameter greater than a diameter of the shank of the neck section and being connected to a center area of the neck section, the stop end portion of the lower semi-spherical portion has an external peripheral portion forming a circular stop surface, and the non-scratching section has an overall diameter greater than a diameter of the stop end portion, wherein the wound covering assembly includes a protective covering member including a covering portion to fitly cover a local wound within a patient's oral cavity, and a TAD including a head and an external threaded shank, wherein a first locating section and a second locating section are provided in opposite side portions of the covering portion, and a bottom surface of the covering portion is an irregular surface matching a shape of the patient's oral cavity, and wherein the external threaded shank extends through the second locating section, the head includes a contact end portion that is pressed against the protective covering member, and the protective covering member is maintained in place by the first locating section and the TAD to cover the wound within the patient's oral cavity.

In one operational embodiment, the opening provided to the engaging end portion of the non-scratching TAD may include a rounded corner portion and a bore portion having a cross-sectional area less than a cross-sectional area of the rounded corner portion, the neck section of the non-scratching TAD further includes an expansion portion connected between the shank of the neck section and the stop end portion of the lower semi-spherical portion, and the expansion portion has a funnel shape having a diameter gradually increasing toward the lower semi-spherical portion.

The protective covering member may include a medical-grade silicon material, another TAD extends through the first locating section, two TADs together maintains the protective covering member in place above the local wound within the patient's oral cavity, and the heads of the two TADs may press the silicon material by pressing areas of the silicon material located in a circumference of the TADs, such that the heads of the TADs are located not higher than an upper surface of the protective covering member.

The free gingival graft kit may further include a locating needle including a handle portion and a prong portion connected to an end portion of the handle portion, wherein the prong portion may include a connecting element and a plurality of prongs apart from each other and mounted on the connecting element, and the prongs may be capable of penetrating the silicon material of the protective covering member to form a plurality of locating points on the protective covering member.

Alternatively, the protective covering member may be formed by three-dimensional (3D) printing using a resin material, the first locating section may be a saw-toothed edge matching a profile of patient's teeth, and the saw-toothed edge may extend toward at least one of a plurality of gaps between the teeth. The second locating section may be formed as a stepped hole including a first opening and a second opening having a diameter greater than a diameter of the first opening, and the head of the TAD may have the height less than a sunken depth of the second opening such that, when the TAD extends toward the stepped hole, the head of the TAD may be completely accommodated in the second opening.

The protective covering member may further include a fixing portion in contact with an external surface of the patient's teeth, and the fixing portion may be a corrugated wall integrally extending upwards from the saw-toothed edge of the first locating section. The covering portion of the protective covering member may include a first portion region in contact with the patient's oral cavity and a second portion region lifted from the first portion region, a gap may be formed between the second portion region and the patient's oral cavity, and the second portion region may be located between the fixing portion and the first portion region.

In another operational embodiment, a free gingival graft kit according to an embodiment further include a suture winding assembly and an elastic covering film in addition to the non-scratching TAD and the wound covering assembly. The suture winding assembly may include a healing abutment including an upper end portion, a downward tapered neck portion, and a bolt portion, which are sequentially arranged from an upper end to a lower end, the upper end portion having an exposed upper end surface and a peripheral surface, and a coupling slot being formed in the exposed upper end surface, and a hood including an elastic material and including a cover portion to contact the exposed upper end portion of the healing abutment and a gripping portion to encompass a circumference of the peripheral surface of the upper end portion, the gripping portion having an inner loop size smaller than a size of an external profile of the healing abutment, wherein, when the hood is fitted over the healing abutment, the gripping portion and the cover portion of the hood may elastically expand to increase the inner loop size of the gripping portion, such that the gripping portion provides a stop step to a circumference of a peripheral surface of the healing abutment to firmly grip the healing abutment.

In one operational embodiment, the hood of the suture winding assembly may integrally extend from the cover portion and may further include a limiting portion having a structure matching a structure of the coupling slot. As the limiting portion is inserted into the coupling slot so that the hood is fitted over the healing abutment, the healing abutment and the hood may be prevented from moving relative to each other.

The free gingival graft kit may further include an elastic covering film having an elastically expandable through-hole, wherein the elastically expandable through-hole may have a diameter less than a diameter of the non-scratching section of the non-scratching TAD, and when the elastic covering film is placed on the non-scratching TAD and moved from the non-scratching section to the neck section, the elastically expandable through-hole may be first expanded from an original size to an expanded size in the non-scratching section and then, while moving to the neck section, may be elastically restored from the expanded size to the original size.

The inventive free gingival graft kit can be used in a free gingival graft method that includes a recipient region mucous incision operation, a recipient region mucous fixing operation, a gingival graft collecting operation, a wound covering and pressing operation, a free gingival graft operation, and a suture stitching operation. In the recipient region mucous incision operation, a target position at a boundary between a patient's gingiva and a mucous may be incised to form a linear wound, and the mucous may be pulled in a tooth root direction so that the linear wound is deformed to a large-sized recipient region wound. In the recipient region mucous fixing operation, the non-scratching TAD may be screwed to the recipient region wound by using a dental handpiece, and the mucous pulled downwards may be supported in the neck section of the non-scratching TAD and located at a certain distance from the patient's gingival.

In the gingival graft collecting operation, a piece of epithelium inside the patient's palate may be incised to collect a piece of the gingiva-containing graft, and the area inside the patient's palate where epithelium is removed may form the local wound. In the wound covering and pressing operation, the protective covering member may cover the local wound, and the saw-toothed edge of the protective covering member may extend to at least one of a plurality of gaps between a plurality of teeth located adjacent to the local wound. Next, the protective covering member may be fixed to the patient's palate by using the TADs to thus cover the entire area of the local wound 51 and provide compression hemostasis to and around the local wound. In the free gingival graft operation, the collected graft may be arranged in the recipient region wound.

In the suture stitching operation, a tail end of a first suture may be fixed to the patient's gingiva, the first suture may be wound around the non-scratching TAD, and then a leading end of the first suture may be also fixed to the patient's gingiva, and thus, the first suture shows a U shape pressing a left region of the gingiva-containing graft, and the graft is attached to the recipient region and blood is well supplied so that wound healing is well performed. Alternatively, after wound around the non-scratching TAD, the suture may be additionally and sequentially round around a wound healing abutment covered by the hood and another auxiliary non-scratching TAD. Lastly, the leading end of the suture may be fixed to the patient's gingiva, and the suture may show a W shape outside the graft.

In another embodiment, the free gingival graft method may further include an elastic covering film attachment operation, a protective covering member removal operation, an elastic covering film removal operation, and a suture and TAD control operation. In the elastic covering film attachment operation, the elastic covering film may be attached to the non-scratching TAD, and the elastic hole in the elastic covering film may be elastically expanded by the non-scratching section of the non-scratching TAD. After the elastic covering film is moved to the neck section of the non-scratching TAD, the elastic hole may be restored to the original size. In this state, the elastic covering film may be maintained in place between the patient's gingiva and the non-scratching TAD.

In the protective covering member removal operation, when the local wound heals and the palate epithelium is regenerated, the TADs may be removed from the patient's palate, and the protective covering member may be separated from the regenerated palate epithelium. In the elastic covering film removal operation, when the recipient region wound with the graft heals and the gingiva is regenerated, the elastic covering films maintained in the neck section of the non-scratching TAD may be pulled externally to pass through the non-scratching section and may be separated from the non-scratching TAD. In the suture and TAD removal operation, the sutures and the non-scratching TADs may be removed from the outside and inside of the regenerated gingival.

### BRIEF DESCRIPTION OF THE DRAWINGS

Exemplary embodiments of the inventive concept will be more clearly understood from the following detailed description taken in conjunction with the accompanying drawings in which:
FIG. 1 schematically illustrates a method, not part of the invention, of collecting a piece of graft for treatment of a wound in a patient's gingiva in existing free gingival graft surgery;
FIG. 2 schematically illustrates a method, not part of the invention, of arranging a collectec graft in the wound in a patient's gingiva in the existing free gingival graft surgery;
FIG. 3 illustrates some basic tools in a free gingival graft kit according to an exemplary embodiment of the invention;
FIG. 4 illustrates some additional auxiliary tools in a free gingival graft kit according to an additional embodiment;
FIG. 5 is a flowchart showing operations of the free gingival graft method according to an embodiment not part of the invention;
FIG. 6 is a cross-sectional view of a non-scratching temporal anchorage device (non-scratching TAD) in the free gingival graft kit of FIG. 3;
FIG. 7 is a plan view of an assembled wound covering assembly according to an embodiment in the free gingival graft kit of FIG. 3;
FIG. 8 is a cross-sectional view of the wound covering assembly of FIG. 7 being fixed within a patient's oral cavity;
FIG. 9 is a cross-sectional view of an assembled suture winding assembly according to the embodiment in the additional auxiliary tools of a free gingival graft kit according to an embodiment, showing that a hood thereof elastically expands and covers over a healing abutment of the suture winding assembly;
FIG. 10 is a cross-sectional view of an assembled suture winding assembly according to another embodiment; and FIG. 11 is an exploded view of a wound covering assembly according to another embodiment;
FIGS. 12A and 12B illustrate auxiliary locating needles according to two different embodiments to be used for free gingival graft surgery;
FIG. 13 visually illustrates a recipient region mucous incision operation of the free gingival graft method according to an embodiment which is not part of the invention:
FIG. 14 visually illustrates a recipient region mucous fixing operation of the free gingival graft method according to an embodiment which is not part of the invention;
FIG. 15 visually illustrates a gingival graft collecting operation of the free gingival graft method according to an embodiment which is not part of the invention;
FIG. 16 visually illustrates a wound covering and pressing operation of the free gingival graft method according to an embodiment which is not part of the invention;
FIG. 17 visually illustrates a free gingival graft operation of the free gingival graft method according to an embodiment which is not part of the invention;
FIG. 18 visually illustrates a suture stitching operation of the free gingival graft method according to an embodiment;
FIG. 19 visually illustrates a stitching sutures operation of a free gingival graft method according to another embodiment which is not part of the invention;
FIG. 20 visually illustrates an elastic covering film attachment operation of the free gingival graft method according to an embodiment which is not part of the invention;
FIG. 21 visually illustrates a protective covering member removal operation of the free gingival graft method according to an embodiment which is not part of the invention;
FIG. 22 visually illustrates an elastic covering film removal operation of the free gingival graft method according to an embodiment which is not part of the invention;
FIG. 23 visually illustrates a suture and TAD removal operation of the free gingival graft method according to an embodiment which is not part of the invention;
FIGS. 24A to 24D visually illustrate a use of a silicon protective covering member in a wound covering and pressing operation to cover a local wound; and
FIG. 25 is a bottom view of a local wound covered by a piece of a silicon protective covering member according to an embodiment.

### DETAILED DESCRIPTION OF THE EXEMPLARY EMBODIMENTS

Hereinafter, the inventive concept will be described in detail by explaining preferred embodiments of the inventive concept with reference to the attached drawings. For easy understanding, like reference numerals denote like elements in the embodiments.

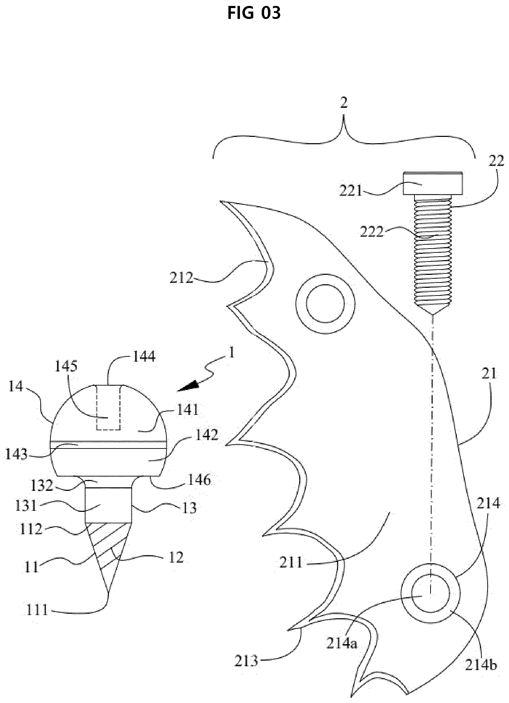

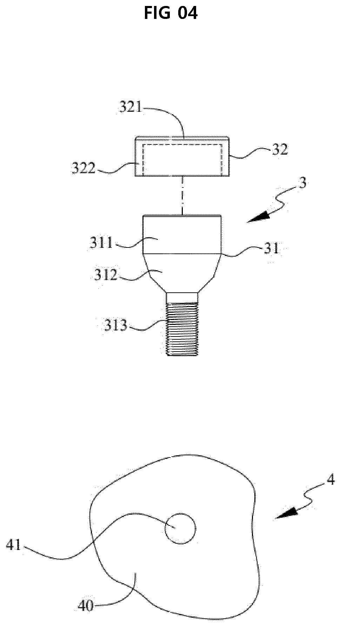

The inventive concept relates to a free gingival graft kit. Referring to FIGS. 3 and 4, the free gingival graft kit includes a non-scratching temporal anchorage device (non-scratching TAD) 1, a wound covering assembly 2, a suture winding assembly 3, and an elastic covering film 4. While the non-scratching TAD 1 and the wound covering assembly 2 are basic tools of the free gingival graft kit, the suture winding assembly 3 and the elastic covering film 4 are additional auxiliary tools of the free gingival graft kit.

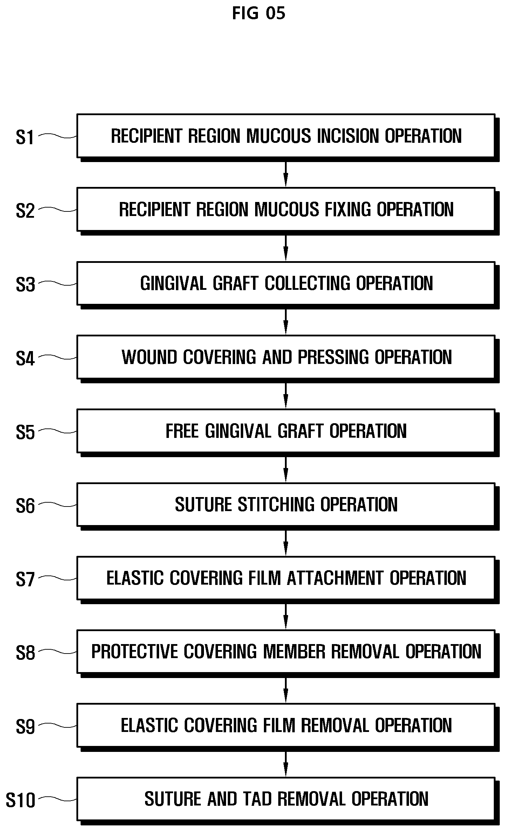

Referring to FIG. 5, a free gingival graft method includes a recipient region mucous incision operation S1, a recipient region mucous fixing operation S2, a gingival graft collecting operation S3, a wound covering and pressing operation S4, a free gingival graft operation S5, a suture stitching operation S6, an elastic covering film attachment operation S7, a protective covering member removal operation S8, an elastic covering film removal operation S9, and a suture and TAD removal operation S10.

The non-scratching TAD 1 may be used in the recipient region mucous fixing operation S2 and the suture stitching operation S6; the wound covering assembly 2 may be used in the wound covering and pressing operation S4; the suture winding assembly 3 may be selectively used in the suture stitching operation S6; and the elastic covering film 4 may be used in the elastic covering film attachment operation S7. As illustrated in FIG. 5, in a flowchart of the free gingival graft method, a list order of the operations S1 to S10 is only an example for convenience of explanation. In an actual implementation of the method, the gingival graft collecting operation S3 and the wound covering and pressing operation S4 may be alternatively performed prior to the recipient region mucous incision operation S1 and the recipient region mucous fixing operation S2. Furthermore, the protective covering member removal operation S8 may be performed after the elastic covering film removal operation S9 and the suture and TAD removal operation S10.

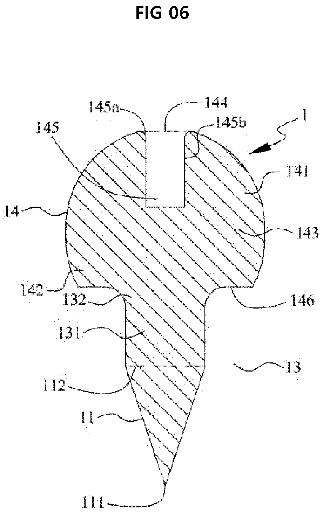

Referring to FIGS. 3 and 6, the non-scratching TAD 1 includes a drilling section 11, a male thread 12, a neck section 13 located at the rear of the drilling section 11, and a non-scratching section 14 located at the rear of the neck section 13. The drilling section 11 includes a pointed tip 111 and an expanded end portion 112, which define together a conical surface therebetween, and the conical surface has an axial length between 3 mm and 5 mm. The male thread 12 is provided on the circumference of the conical surface of the drilling section 11, thereby allowing the drilling section 11 to be firmly fixed to a patient's alveolar bone. The neck section 1 has a length between 3 mm and 4 mm, and includes a shank 131 connected to the expanded end portion 112 of the drilling section 11 and an expansion portion 132 connected to the shank 131. The shank 131 has a smooth external surface, and the expansion portion 132 has a funnel shape with a diameter increasing rearwards.

The non-scratching section 14 includes an upper semi-spherical portion 141, a lower semi-spherical portion 142, and a circular disc portion 143 located between the upper semi-spherical portion 141 and the lower semi-spherical portion 142. The upper semi-spherical portion 141 has an engaging end portion 144 to which a handpiece is engaged. An opening 145 that does not include a sharp corner is provided in the engaging end portion 144. As illustrated in FIGS. 3 and 6, the opening 145 includes a rounded corner portion 145a located in an upper end of the opening 145, and a bore portion 145b having a cross-sectional area less than a cross-sectional area of the rounded corner portion 145a and having a polygonal shape for engagement with a dental handpiece. The lower semi-spherical portion 142 includes a stop end portion 146 having a diameter greater than the diameter of the shank 131 of the neck section 13. The stop end portion 146 has an external peripheral portion forming a circular stop surface to stop a center area connected to the neck section 13 and the suture from moving toward the upper semi-spherical portion 141 across the lower semi-spherical portion 142. Furthermore, the non-scratching section 14 has an overall diameter greater than the diameter of the stop end portion 146.

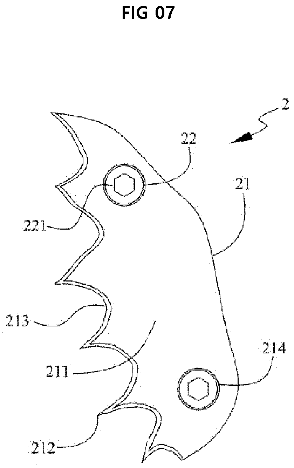

Referring to FIGS. 3, 7, and 8, in an embodiment of the inventive concept, the wound covering assembly 2 includes a protective covering member 21 and a plurality of temporal anchorage devices (TADs) 22. The protective covering member 21 may formed by three-dimensional (3D) printing using a resin material corresponding to the shape of a patient's oral cavity obtained through 3D scanning, so that the protective covering member 21 may be fitly attached to a patient's palate, a gum, and a plurality of teeth 50 within a patient's oral cavity. The protective covering member 21 includes a covering portion 211 having an area greater than an area of a wound within a patient's oral cavity, a fixing portion 212 contacting the outside of the teeth 50 located adjacent to a local wound 51 from which a piece of a gingiva-containing graft 73 is collected, and first and second locating sections 213 and 214 located at both side portions of the covering portion 211 facing each other.

The first locating section 213 is a saw-toothed edge located at one side portion of the covering portion 211 and matching the profile of the teeth 50, and allows the saw-toothed edge to extend to at least one of a plurality of gaps of the teeth 50. The second locating section 214 includes two stepped holes provided in the covering portion 211. However, the number of the covering portion 211 described above is an example, and the disclosure is not limited thereto. Furthermore, the covering portion 211 has a bottom surface that is an irregular surface 215 matching the patient's oral cavity shape. The fixing portion 212 integrally extends upwards from the saw-toothed edge, thereby providing a corrugated wall.

Each of the TADs 22 includes a flat head 221 and an external threaded shank 222. When the protective covering member 21 is arranged within a patient's oral cavity corresponding to the teeth 50 and the local wound 51, the external threaded shanks 222 extend through the stepped holes of the second locating section 214, the flat heads 221 may each have a contact end portion pressed against the protective covering member 21. In this state, both side portions of the protective covering member 21 are fixed in place by the saw-toothed edge of the first locating section 213 and the TADs 22 in the two stepped holes of the second locating section 214, and thus, the protective covering member 21 applies a pressing force F to the local wound 51 so as to facilitate hemostasis in the local wound 51.

As illustrated in FIG. 3, in an embodiment of the inventive concept, each of the stepped holes of the second locating section 214 includes a first opening 214a and a second opening 214b. The second opening 214b has a diameter greater that the diameter of the first opening 214a. Furthermore, the flat head 221 of each of the TADs 22 has a height less than a sunken depth of the second opening 214b. When the TADs 22 extend into the stepped holes, the flat heads 221 of the TADs 22 are completely accommodated in the second opening 214b and have the same height as an external end portion surface of the second opening 214b. Accordingly, the protective covering member 21 and the TADs 22 together form a continuous surface.

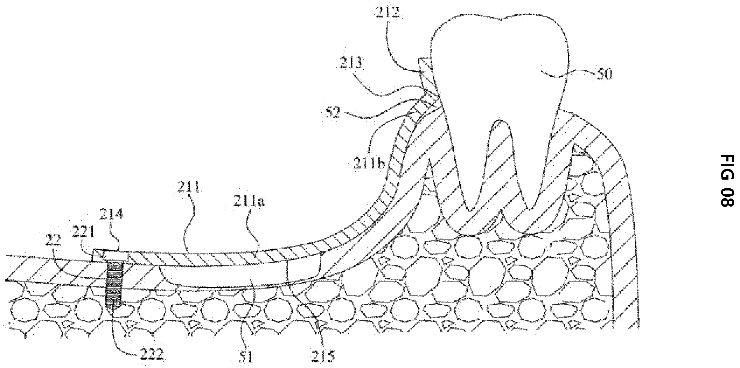

As can be seen in FIG. 8, the covering portion 211 includes a first portion region 211a in contact with patient's oral cavity and a second portion region 211b lifted from the first portion region 211a, and thus, a gap 52 is formed between the second portion region 211b and the patient's oral cavity. In detail, the second portion region 211b is located between the fixing portion 212 and the first portion region 211a to be adjacent thereto.

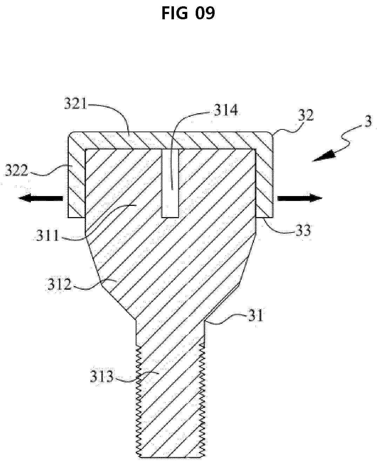

Referring to FIGS. 4 and 9, the suture winding assembly 3 includes a healing abutment 31 and a hood 32. The healing abutment 31 includes an upper end portion 311, a downward tapered neck portion 312, and a bolt portion 313, which are sequentially arranged in the healing abutment 31 from an upper end to a lower end. The upper end portion 311 has an exposed upper end surface and a peripheral surface. A coupling slot 314 is formed in the exposed upper end surface of the upper end portion 311. The hood 32 is made of an elastic material, such as silicon or rubber, and includes a cover portion 321 to contact the exposed upper end surface of the upper end portion 311 and a gripping portion 322 surrounding the circumference of a peripheral portion of the upper end portion 311. The gripping portion 322 has an inner loop size smaller than an external profile of the healing abutment 31. When the hood 32 is fitted over the healing abutment 31, the gripping portion 322 and the cover portion 321 of the hood 32 are elastically expanded to increase the inner loop size of the gripping portion 322, and thus, the gripping portion 322 provides a stop step 33 to the circumference of a peripheral surface of the healing abutment 31 to firmly grip the healing abutment 31.

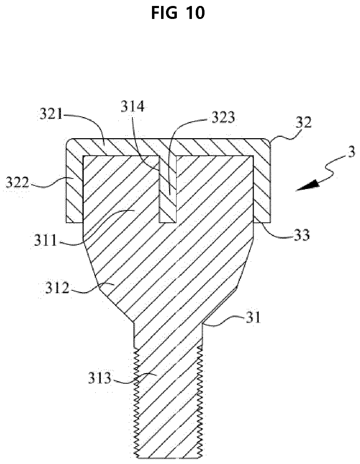

Referring to FIG. 10, in the suture winding assembly 3 of another embodiment, the hood 32 further includes a limiting portion 323 integrally extending from the cover portion 321 and having a structure matching the structure of the coupling slot 314. When the limiting portion 323 is inserted into the coupling slot 314 so that the hood 32 is fitted over the healing abutment 31, the healing abutment 31 and the hood 32 are prevented from moving relative to each other.

Referring back to FIG. 4, the elastic covering film 4 includes a piece of a large-sized film 40 having an elastically expandable through-hole 41. The elastically expandable through-hole 41 has a diameter less than that diameter of the non-scratching section 14 of the non-scratching TAD 1. When the elastic covering film 4 is placed on the non-scratching TAD 1 and moved from the non-scratching section 14 to the neck section 13, the elastically expandable through-hole 41 is first extended from the original size to an expanded size in the non-scratching section 14 and then, while moved to the neck section 13, elastically restored to the original size from the expanded size, thereby maintained between the stop end portion 146 of the non-scratching TAD 1 and the patient's gingiva. In another operational embodiment, the elastic covering film 4 has two elastically expandable through-holes 41 formed to be apart from each other by a predetermined distance. In this manner, a single piece of the elastic covering film 4 may be located in place in two opposite end portions by using two pieces of the non-scratching TADs 1. The use of the elastic covering film 4 prevents the non-scratching TAD 1 from being completely encompassed by patient's regenerated gingiva, and also prevents a situation in which the non-scratching TAD 1 is not easily found after a surgery wound heals.

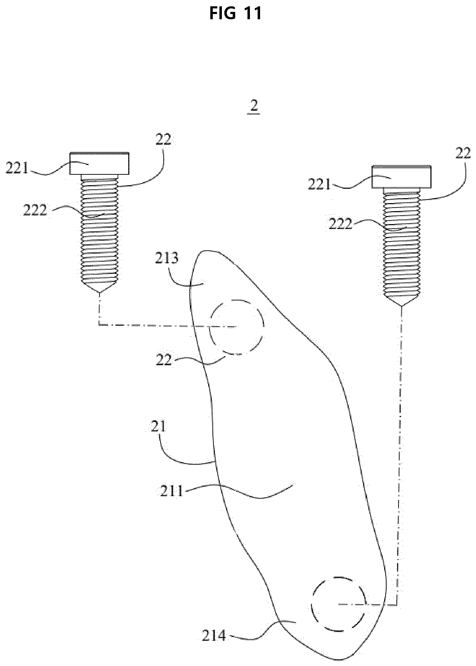

Referring to FIG. 11, in the wound covering assembly 2 of another embodiment, the protective covering member 21 is made of a medical-grade material that is deformable, such as a silicon sheet that is easily penetrated, so that the protective covering member 21 is automatically deformable to the irregular surface 215 to be fitly attached to the local wound 51 and peripheral tissues thereof. In another embodiment, the protective covering member 21 also includes the covering portion 211 having an area slightly greater than the area of a local wound. Some areas of the protective covering member 21 extend over the local wound 51 by a specific length to provide the first locating section 213 and the second locating section 214, and the TADs 22 pass through the first and second locating sections 213 and 214 in these extended areas.

A surgeon determines a penetration position in each of the first and second locating sections 213 and 214 (se dashed circles), and fixes the two TADs 22 to the protective covering member 21 by using a dental handpiece at the determined penetration positions. However, as the covering portion 211 formed as a silicon sheet tends to slip with respect to the local wound 51, in the process of fixing the TADs 22, to prevent the covering portion 211 of silicon from moving and rotation, there is a need to keep the covering portion 211 in place by using one set of two locating needles 6.

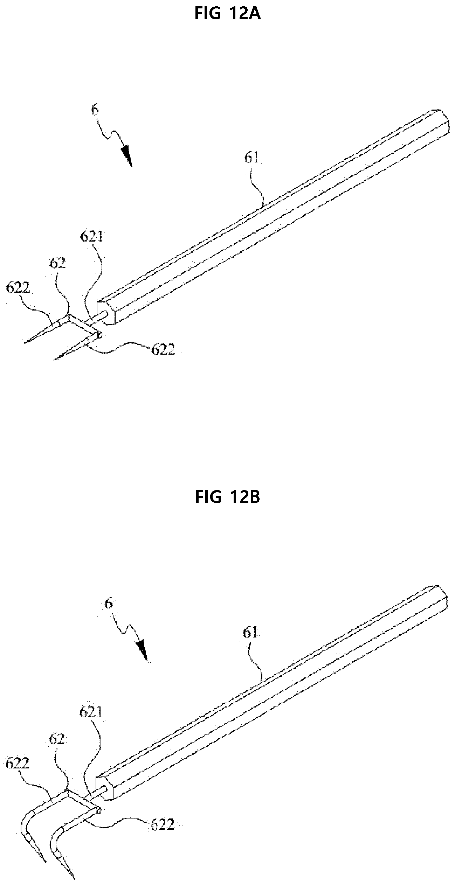

Referring to FIGS. 12A and 12B, the locating needles 6 may each include a handle portion 61 and a prong portion 62 connected to an end portion of the handle portion 61. The prong portion 62 includes a connecting element 621 and a plurality of prongs 622 apart from each other and mounted on the connecting element 621. The prongs 622 penetrates the protective covering member 21 formed of silicon to form a plurality of locating points on the protective covering member 21. As illustrated in FIG. 12A, the connecting element 621 has a T-shaped link form, and the prongs 622 are each a straight prong. The locating needle 6 with the prongs 622 having a straight shape is designed such that a right-handed user can access the upper right area of the protective covering member 21, and a left-handed user can access the upper left area of the protective covering member 21. In another embodiment as illustrated in FIG. 12B, while the connecting element 621 also has a T-shape, the prongs 622 are each an angled prong. The locating needle 6 with the prongs 622 that are angled are designed such that a right-handed user can access the upper left area of the protective covering member 21, and a left-handed user can access the upper right area of the protective covering member 21.

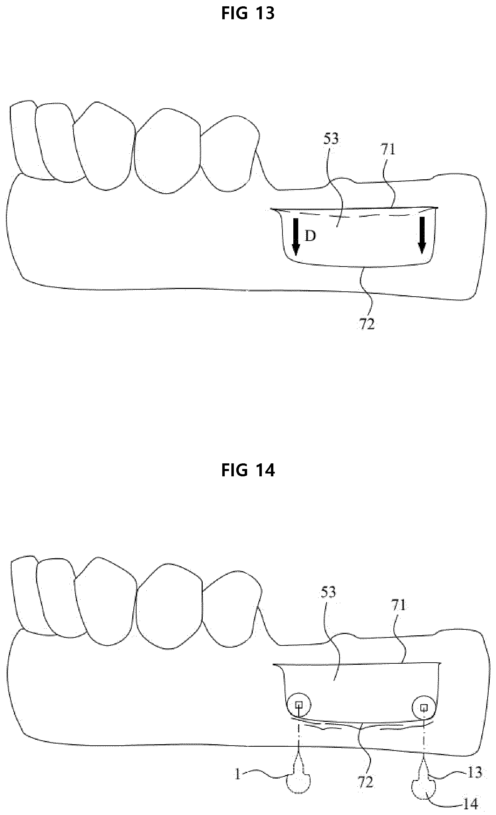

Referring to FIG. 13, in the recipient region mucous incision operation S1 of the free gingival graft method according to an embodiment, a target position at a boundary between a patient's gingiva 71 and a mucous 72 is incised to form a linear wound as indicated by a dashed line, and the mucous 72 is pulled in a tooth root direction D so that the linear wound is deformed to a large-sized recipient region wound 53.

Referring to FIG. 14, in the recipient region mucous fixing operation S2 of the free gingival graft method according to an embodiment, the two non-scratching TADs 1 are screwed, by using a dental handpiece, downwards with respect to the recipient region wound 53 in two opposite end portions of the recipient region wound 53, and thus, the mucous 72 pulled downwards are supported in the neck sections 13 of the two TADs 1 to be located at a certain distance from the patient's gingiva 71. As each TAD 1 has the non-scratching section 14 having a diameter greater than the diameter of the neck section 13, the mucous 72 contacts the stop end portions 146 of the TADs 1 formed between the neck sections 13 and the non-scratching sections 14, and stops moving across the non-scratching sections 14. Accordingly, the mucous 72 that is incised is prevented from being repulsed and deformed.

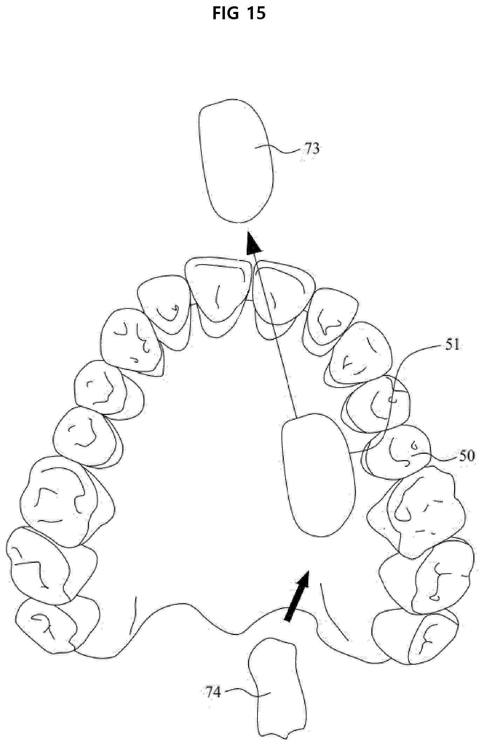

Referring to FIG. 15, in the gingival graft collecting operation S3 of the free gingival graft method according to an embodiment, a piece of epithelium inside the patient's palate is incised to collect a piece of the gingiva-containing graft 73. The area inside the patient's palate where epithelium is removed forms the local wound 51, and later, a treatment medication 74, such as hemostasis gauze or a growth factor, is arranged in the local wound 51.

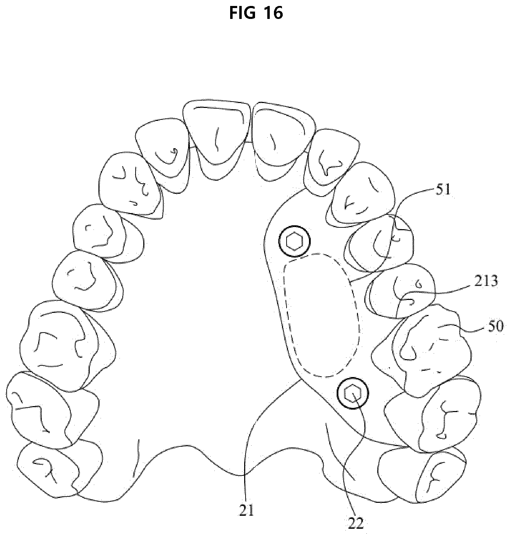

Referring to FIG. 16, in the wound covering and pressing operation S4 of the free gingival graft method according to an embodiment, the protective covering member 21 covers the local wound 51, and the saw-toothed edge 213 of the protective covering member 21 extends to at least one of a plurality of gaps between a plurality of teeth located adjacent to the local wound 51. Next, the protective covering member 21 is fixed to the patient's palate by using the TADs 22 to thus cover the entire area of the local wound 51 and provide compression hemostasis to and around the local wound 51.

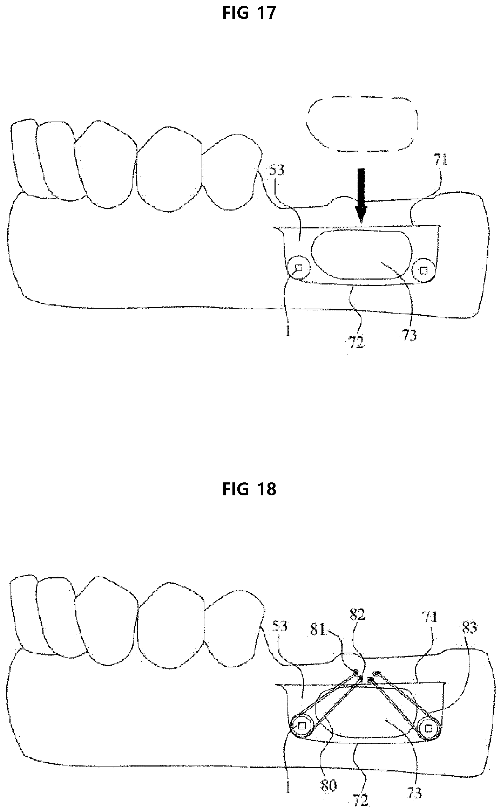

Referring to FIG. 17, in the free gingival graft operation S5 of the free gingival graft method according to an embodiment, the gingiva-containing graft 73 is arranged in the recipient region wound 53. As illustrated in FIG. 18, in the suture stitching operation S6 of the free gingival graft method according to an embodiment, a tail end 81 of a first suture 80 is fixed to the patient's gingiva 71, the first suture 80 is wound around the non-scratching TAD 1 corresponding thereto, and then a leading end 82 of the first suture 80 is also fixed to the patient's gingiva 71. Thus, the first suture 80 shows a U shape pressing a left region of the gingiva-containing graft 73. Next, in the same manner, a second suture 83 is stitched so as to press a right region of the gingiva-containing graft 73. In this manner, the gingiva-containing graft 73 is supported by the first and second sutures 80 and 83 to maintain the contact with the recipient region wound 53.

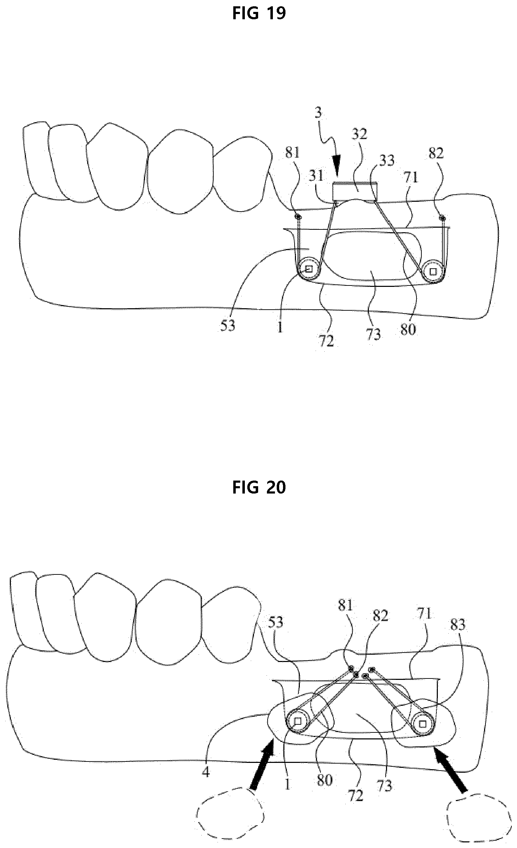

Alternatively, the first suture 80 may be stitched in a different manner. Referring to FIG. 19, after the first suture 80 is wound around the first non-scratching TAD 1, the first suture 80 may be sequentially further wound around the suture winding assembly 3 and another non-scratching TAD 1. Lastly, the leading end 82 of the first suture 80 is fixed to the patient's gingiva 71. In this stitching manner, the first suture 80 is supported in the stop step 33 formed between the healing abutment 31 and the hood 32 of the suture winding assembly 3, finally showing a W shape.

Referring to FIG. 20, in the elastic covering film attachment operation S7 of the free gingival graft method according to an embodiment, the two elastic covering films 4 are respectively attached to the two non-scratching TADs 1. The elastically expandable through-holes 41 formed in the elastic covering films 4 are elastically expanded by the non-scratching sections 14 of the non-scratching TADs 1. After the elastic covering films 4 are moved to the neck sections 13 of the non-scratching TADs 1, the elastically expandable through-hole 41 are restored to the original size. In this state, one of the two elastic covering films 4 is maintained in place between the first suture 80 and the non-scratching section 14 corresponding thereto, whereas another of the elastic covering films 4 is maintained in place between the second suture 83 and another non-scratching section 14 corresponding thereto.

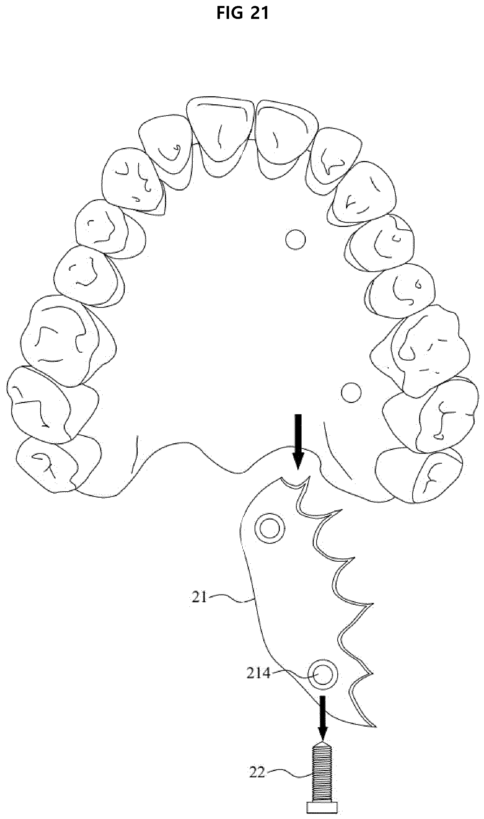

Referring to FIG. 21, in the protective covering member removal operation S8 of the free gingival graft method according to an embodiment, when the local wound 51 heals and the palate epithelium is regenerated, the TADs 22 are removed from the patient's palate and the stepped holes of the second locating section 214, and the protective covering member 21 is separated from the regenerated palate epithelium so as to heal small wounds generated by the TADs 22.

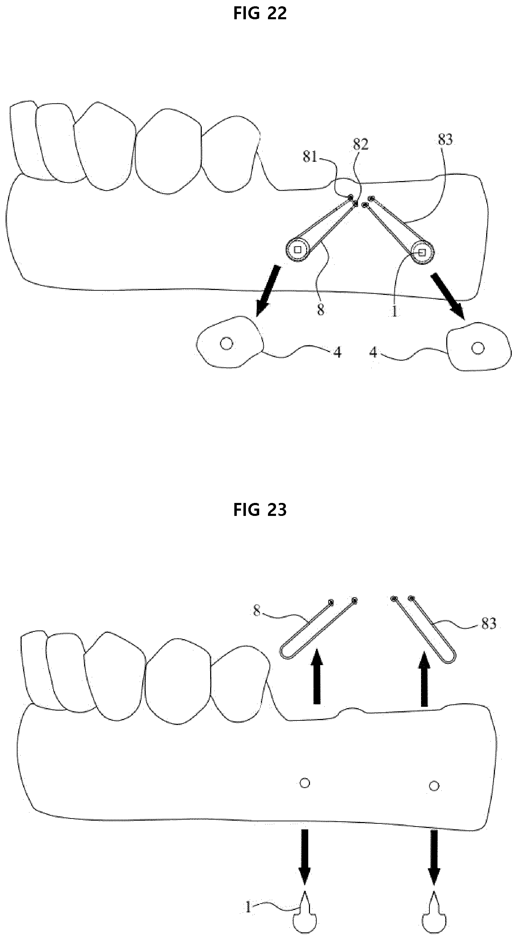

Referring to FIG. 22, in the elastic covering film removal operation S9 of the free gingival graft method according to an embodiment, the recipient region wound 53 covered with the gingiva-containing graft 73 heals and gingiva is regenerated, the elastic covering films 4 maintained in the neck sections 13 of the non-scratching TADs 1 are pulled externally to pass through the non-scratching sections 14 and to be separated from the non-scratching TADs 1. As the elastic covering films 4 are arranged outside the gingiva-containing graft 73 and the recipient region wound 53, the gingiva that heals and is regenerated does not grow externally to encompass the non-scratching sections 14, but is located inside the elastic covering films 4.

Referring to FIG. 23, in the suture and TAD removal operation S10 of the free gingival graft method according to an embodiment, the first suture 80, the second suture 83, and the non-scratching TADs 1 are removed from the outside and inside of the regenerated gingiva, and small openings formed by the non-scratching TADs 1 may gradually heal.

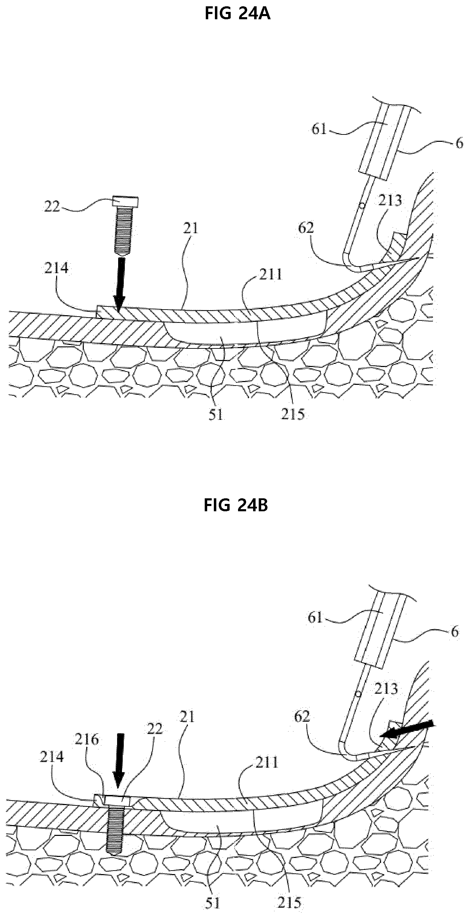

In the wound covering and pressing operation S4, when the protective covering member 21 used to cover the local wound 51 is a silicon sheet, a surgeon may check and determine an actual would state within the patient's oral cavity, and select in advance a silicon sheet having a size greater than the local wound 51 according to the determined wound state. Referring to FIG. 24A, the surgeon places the protective covering member 21 above the local wound 51, and thus, a partial portion of the protective covering member 21 is located outside the local wound 51 to install the TADs 22. The locating needle 6 with the angled prongs 622 pass through the protective covering member 21 to penetrate into the gingiva around the patient's teeth, thereby locating the protective covering member 21 in place. Next, the TADs 22 are aligned with the penetration position of the second locating section 214 to extend therethrough.

Referring to FIG. 24B, when the TAD 22 penetrates into the patient's hard palate through the second locating section 214, an opening is formed in the second locating section 214, and the flat head 221 of the TAD 22 is pressed against the second locating section 214 to press the external peripheral area 216 in a radial direction of the opening. Thus, the flat head 221 of the TAD 22 does not cause inconvenience to the patient's tongue to be the same height as the upper surface of the protective covering member 21.

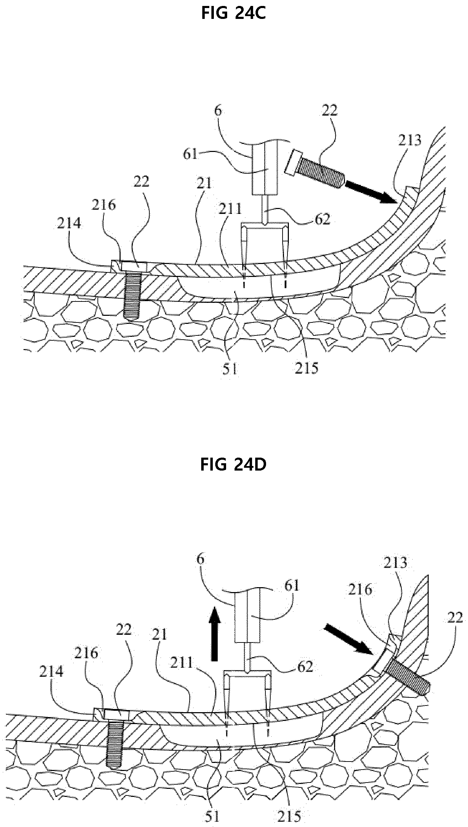

Referring to FIG. 24C, then, the locating needle 6 with the angled prongs 622 is removed from the protective covering member 21, and another locating needle 6 with the prongs 622 in a straight shape is used to penetrate the patient's gingiva around the first locating section 213. Next, another TAD 22 is aligned with the penetration position of the first locating section 213.

Referring to FIG. 24D, when another TAD 22 is penetrated into the patient's hard palate through the first locating section 213, an opening is formed in the first locating section 213. When another TAD 22 is fixed to the patient's hard palate, the locating needle 6 with the prongs 622 having a straight shape is removed from the patient's gingiva.

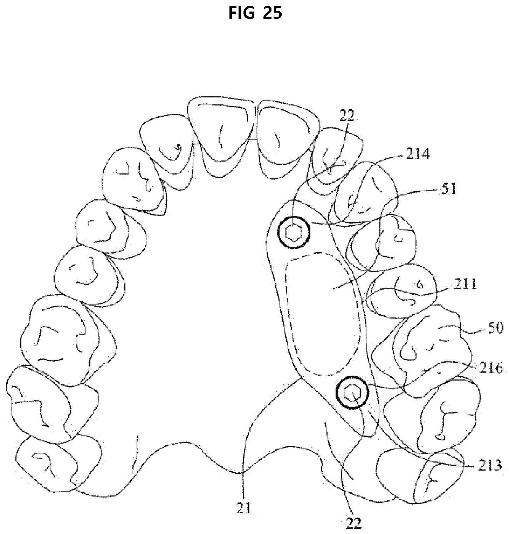

Referring to FIG. 25, as the protective covering member 21 formed of a silicon sheet is easy to penetrate, the protective covering member 21 may be fixed to the local wound 51 by using two TADs 22. A treatment medication (not shown) is arranged between the bottom surface of the protective covering member 21 and the local wound 51 to help hemostasis of the local wound 51 and restrict a movement of blood clots, thereby improving wound healing and minimizing patient's inconvenience during a wound healing time.

The inventive free gingival graft kit can be used as follows: when the incised mucous is pulled downwards, a plurality of non-scratching TADs are used to prevent the mucous from repulsing upwards, thereby allowing a larger area of the recipient region wound to accommodate a graft to be exposed. Accordingly, the demerits of an existing free gingival graft method, in which a lot of time is consumed to stitch the graft to the periosteum by using sutures, and which tends to fail due to periosteum damage. In the meantime, the non-scratching TAD serves as an important locating position around which a suture is wound, and thus, the suture may press the graft against the recipient region wound, and a dentist only needs to stitch a suture to the gingiva without extending the suture through the periosteum. Furthermore, the protective covering member covers a local wound from which the graft is collected, to provide compression hemostasis to the local wound, and prevents the local wound from contacting the patient's tongue or food. Accordingly, the wound quickly heals so that patient's inconvenience may be minimized.

While the inventive concept has been particularly shown and described with reference to exemplary embodiments thereof, it will be understood that various changes in form and details may be made therein, the scope of the invention being defined by the following claims.

## Claims

1. A free gingival graft kit comprising:
- a wound covering assembly (2), comprising
a protective covering member (21) including a covering portion (211) to fitly cover a local wound within a patient's oral cavity; wherein a first locating section (213) and a second locating section (214) are provided in opposite side portions of the covering portion (211), and a bottom surface of the covering portion is an irregular surface (215) matching a shape of the patient's oral cavity, and a Temporal Anchorage Device, TAD, (22) including a head (221) and an external threaded shank (222), wherein, in use, the external threaded shank (222) extends through the second locating section (214), the head (221) includes a contact end portion that is pressed against the protective covering member (21), and the protective covering member (21) is maintained in place by the first locating section (213) and the TAD (22) to cover the wound within the patient's oral cavity; and
- a non-scratching TAD (1) comprising:
a drilling section (11) having a pointed tip (111) and an expanded end portion (112), wherein a conical surface is defined between the pointed tip (111) and the expanded end portion (112);
a male thread (12) provided on a circumference of the conical surface of the drilling section (11);
a neck section (13) connected to the expanded end portion (112) of the drilling section (11) and including a shank (131) having a smooth external surface; and
a non-scratching section (14) including an upper semi-spherical portion (141) and a lower semi-spherical portion (142), wherein the upper semi-spherical portion (141) includes an engaging end portion (144) to be engaged with a dental handpiece, the engaging end portion (144) being provided with an opening (145) that does not include a sharp corner, and wherein the lower semi-spherical portion (142) includes a stop end portion (146), the stop end portion (146) having a diameter greater than a diameter of the shank (131) of the neck section (13) and being connected to a center area of the neck section (13), the stop end portion (146) of the lower semi-spherical portion (142) has an external peripheral portion forming a circular stop surface, and the non-scratching section (14) has an overall diameter greater than a diameter of the stop end portion.

2. The free gingival graft kit of claim 1, wherein the opening (145) provided to the engaging end portion (144) of the non-scratching TAD (1) includes a rounded corner portion (145a) and a bore portion (145b) having a cross-sectional area less than a cross-sectional area of the rounded corner portion (145a),
the neck section (13) of the non-scratching TAD (1) further includes an expansion portion (132) connected between the shank (131) of the neck section (13) and the stop end portion (146) of the lower semi-spherical portion (142), and
the expansion portion (132) has a funnel shape having a diameter gradually increasing toward the lower semi-spherical portion (142).

3. The free gingival graft kit of claim 1, wherein the protective covering member (21) includes a medical-grade silicon material, and wherein another TAD (22) is provided that extends through the first locating section (214), the two TADs (22) together maintain, in use, the protective covering member (21) in place above the local wound within the patient's oral cavity, and
the heads (221) of the two TADs, in use, press the silicon material by pressing areas of the silicon material located in a circumference of the TADs (22), such that the heads of the TADs (221) are located not higher than an upper surface of the protective covering member (21).

4. The free gingival graft kit of claim 3, further comprising a locating needle (6) including a handle portion (61) and a prong portion (62) connected to an end portion of the handle portion (61), wherein the prong portion (62) includes a connecting element (621) and a plurality of prongs (622) apart from each other and mounted on the connecting element (621), and
the prongs (622) are capable of penetrating the silicon material of the protective covering member (21) to form a plurality of locating points on the protective covering member (21).

5. The free gingival graft kit of claim 1, wherein the protective covering member (21) is formed by three-dimensional (3D) printing using a resin material, the first locating section (213) is a saw-toothed edge matching a profile of patient's teeth, and, in use, the saw-toothed edge extends toward at least one of a plurality of gaps between the teeth.

6. The free gingival graft kit of claim 5, wherein the second locating section (214) is formed as a stepped hole including a first opening (214a) and a second opening (214b) having a diameter greater than a diameter of the first opening, and
the head (221) of the TAD (22) has a height less than a sunken depth of the second opening (214b) such that, when the TAD (22) extends toward the stepped hole, the head (221) of the TAD (22) is completely accommodated in the second opening (214b).

7. The free gingival graft kit of claim 5, wherein the protective covering member (21) further includes a fixing portion (212) that is, in use, in contact with an external surface of the patient's teeth, and the fixing portion (212) is a corrugated wall integrally extending upwards from the saw-toothed edge of the first locating section (213).

8. The free gingival graft kit of claim 7, wherein the covering portion (211) of the protective covering member (21) includes a first portion region (211a) that, in use, is in contact with the patient's oral cavity and a second portion region (211b) lifted from the first portion region (211a), in use, a gap is formed between the second portion region (211b) and the patient's oral cavity, and the second portion region (211b) is located between the fixing portion (212) and the first portion region (211a).

9. The free gingival graft kit of claim 1, further comprising a suture winding assembly (3),
wherein the suture winding assembly (3) comprises:
a healing abutment (31) including an upper end portion (311), a downward tapered neck portion (312), and a bolt portion (313), which are sequentially arranged from an upper end to a lower end, the upper end portion (311) having an exposed upper end surface and a peripheral surface, and a coupling slot (314) being formed in the exposed upper end surface; and
a hood (32) including an elastic material and including a cover portion (321) to contact the exposed upper end portion (311) of the healing abutment (31) and a gripping portion (322) to encompass a circumference of the peripheral surface of the upper end portion (311), the gripping portion (322) having an inner loop size smaller than a size of an external profile of the healing abutment (31),
wherein, when the hood (32) is fitted over the healing abutment (31), the gripping portion (322) and the cover portion (321) of the hood (32) elastically expand to increase the inner loop size of the gripping portion (322), such that the gripping portion (322) provides a stop step (33) to a circumference of a peripheral surface of the healing abutment (31) to firmly grip the healing abutment (31).

10. The free gingival graft kit of claim 1, further comprising an elastic covering film (4) having an elastically expandable through-hole (41),
wherein the elastically expandable through-hole (31) has a diameter less than a diameter of the non-scratching section (14) of the non-scratching TAD (1), and
when the elastic covering film (4) is placed on the non-scratching TAD (1) and moved from the non-scratching section (14) to the neck section (13), the elastically expandable through-hole (41) is first expanded from an original size to an expanded size in the non-scratching section (14) and then, while moving to the neck section (13), is elastically restored from the expanded size to the original size.

## Patentansprüche

1. Freier Zahnfleischtransplantatsatz, der umfasst:
eine Wundabdeckungsanordnung (2), die umfasst ein Schutzabdeckungselement (21), das einen Abdeckungsabschnitt (211) enthält, um eine lokale Wunde innerhalb einer Mundhöhle eines Patienten angemessen zu bedecken; wobei ein erster Lageabschnitt (213) und ein zweiter Lageabschnitt (214) in gegenüberliegenden Seitenabschnitten des Abdeckungsabschnitts (211) bereitgestellt sind,
und wobei eine untere Oberfläche des Abdeckungsabschnitts eine unregelmäßige Oberfläche (215) ist, die auf eine Form der Mundhöhle des Patienten passend abgestimmt ist, und
eine temporäre Verankerungsvorrichtung (22), TAD (Temporal Anchorage Device), umfasst, die einen Kopf (221) und einen äußeren Gewindeschaft (222) enthält, wobei sich während der Anwendung der äußere Gewindeschaft (222) durch den zweiten Lageabschnitt (214) erstreckt, wobei der Kopf (221) einen Kontaktendabschnitt enthält, der gegen das Schutzabdeckungselement (21) gedrückt ist, und wobei das Schutzabdeckungselement (21) durch den ersten Lageschnitt (213) und die TAD-Vorrichtung (22) an Ort und Stelle gehalten wird, um die Wunde innerhalb der Mundhöhle des Patienten abzudecken; und
eine kratzfreie TAD-Vorrichtung (1), die umfasst:
ein Bohrabschnitt (11) mit einer spitz zulaufenden Spitze (111) und mit einem erweiterten Endabschnitt (112), wobei zwischen der spitz zulaufenden Spitze (111) und dem erweiterten Endabschnitt (112) eine konische Oberfläche definiert ist;
ein Außengewinde (12), das an einem Umfang der konischen Fläche des Bohrabschnitts (11) bereitgestellt ist;
einen Halsabschnitt (13), der mit dem erweiterten Endabschnitt (112) des Bohrabschnitts (11) verbunden ist, und der einen Schaft (131) mit einer glatten Außenfläche enthält;
einen kratzfreien Abschnitt (14), der einen oberen halbkugelförmigen Abschnitt (141) und einen unteren halbkugelförmigen Abschnitt (142) enthält, wobei der obere halbkugelförmige Abschnitt (141) einen Eingriffsendabschnitt (144) enthält, um mit einem zahnärztlichen Handstück in Eingriff zu gelangen, wobei der Eingriffsendabschnitt (144) mit einer Öffnung (145) versehen ist, die keine scharfe Ecke enthält, und wobei der untere halbkugelförmige Abschnitt (142) einen Endanschlagsabschnitt (146) enthält, wobei der Endanschlagsabschnitt (146) einen Durchmesser aufweist, der größer als ein Durchmesser des Schafts (131) des Halsabschnitts (13) ist und mit einem mittleren Bereich des Halsabschnitts (13) verbunden ist, wobei der Endanschlagsabschnitt (146) des unteren halbkugelförmigen Abschnitts (142) einen äußeren Randabschnitt aufweist, der eine kreisförmige Anschlagsfläche bildet, und wobei der kratzfreie Abschnitt (14) einen Gesamtdurchmesser aufweist, der größer als ein Durchmesser des Endanschlagsabschnitts ist.

2. Freier Zahnfleischtransplantatsatz nach Anspruch 1, wobei die Öffnung (145), die dem Eingriffsendabschnitt (144) der kratzfreien TAD-Vorrichtung (1) zugeordnet ist, einen abgerundeten Eckabschnitt (145a) und einen Bohrungsabschnitt (145b) enthält, dessen Querschnittsfläche kleiner als eine Querschnittsfläche des abgerundeten Eckabschnitts (145a) ist,
wobei der Halsabschnitt (13) der kratzfreien TAD-Vorrichtung (1) ferner einen Erweiterungsabschnitt (132) enthält, der zwischen dem Schaft (131) des Halsabschnitts (13) und dem Endanschlagsabschnitt (146) des unteren halbkugeligen Abschnitts (142) verbunden ist, und
wobei der Erweiterungsabschnitt (132) eine Trichterform mit einem Durchmesser aufweist, der allmählich in Richtung des unteren halbkugeligen Abschnitts (142) ansteigt.

3. Freier Zahnfleischtransplantatsatz nach Anspruch 1, wobei das Schutzabdeckungselement (21) ein medizinisches Siliconmaterial enthält, und wobei eine andere TAD-Vorrichtung (22) bereitgestellt ist, die sich durch den ersten Lageabschnitt (214) erstreckt, wobei die zwei TAD-Vorrichtungen (22) zusammen in der Anwendung das Schutzabdeckungselement (21) über der örtlichen Wunde innerhalb der Mundhöhle des Patienten an Ort und Stelle halten, und wobei die Köpfe (221) der zwei TAD-Vorrichtungen in der Anwendung das Siliconmaterial durch Drücken von Bereichen des Siliconmaterials, das sich am Umfang der TAD-Vorrichtungen (22) befindet, derart pressen, dass die Köpfe der TAD-Vorrichtungen (221) nicht höher als eine Oberseite des Schutzabdeckungselements (21) angeordnet sind.

4. Freier Zahnfleischtransplantatsatz nach Anspruch 3, der ferner eine Ortungsnadel (6) umfasst, die einen Griffabschnitt (61) und einen Zackenabschnitt (62) enthält, der mit einem Endabschnitt des Griffabschnitts (61) verbunden ist, wobei der Zackenabschnitt (62) ein Verbindungselement (621) und eine Vielzahl von Zacken (622) umfasst, die getrennt voneinander angeordnet und auf dem Verbindungselement (621) befestigt sind, und
wobei die Zacken (622) in der Lage sind, das Silikonmaterial des Schutzabdeckungsabschnitts (21) zu durchdringen, um eine Vielzahl von Ortungspunkten auf dem Schutzabdeckungsabschnitt (21) zu bilden.

5. Zahnfleischtransplantatsatz nach Anspruch 1, wobei das Schutzabdeckungselement (21) durch einen dreidimensionalen (3D) Druck unter Verwendung eines Harzmaterials hergestellt wird, wobei der erste Lageabschnitt (213) eine sägezahnförmige Kante bildet, die einem Profil der Zähne des Patienten entspricht, und wobei sich in der Anwendung die sägezahnförmige Kante auf mindestens eine von mehreren Lücken zwischen den Zähnen erstreckt.

6. Freier Zahnfleischtransplantatsatz nach Anspruch 5, wobei der zweite Lageabschnitt (214) als ein abgestuftes Loch ausgebildet ist, das eine erste Öffnung (214a) und eine zweite Öffnung (214b) mit einem Durchmesser, der größer als ein Durchmesser der ersten Öffnung ist, enthält, und
wobei der Kopf (221) der TAD-Vorrichtung (22) eine Höhe, die kleiner als eine versenkte Tiefe der zweiten Öffnung (214b) ist, derart aufweist, dass dann, wenn sich die TAD-Vorrichtung (22) in Richtung des gestuften Lochs bewegt, der Kopf (221) der TAD-Vorrichtung (22) vollständig in der zweiten Öffnung (214b) untergebracht ist.

7. Freier Zahnfleischtransplantatsatz nach Anspruch 5, wobei das Schutzabdeckungselement (21) ferner einen Befestigungsabschnitt (212) umfasst, der während der Anwendung mit einer Außenfläche der Zähne des Patienten in Kontakt steht, und wobei der Befestigungsabschnitt (212) eine gewellte Wand ist, die sich von der sägezahnförmigen Kante des ersten Lageabschnitts (213) einstückig nach oben erstreckt.

8. Freier Zahnfleischtransplantatsatz nach Anspruch 7, wobei der Abdeckungsabschnitt (211) des Schutzabdeckungselements (21) einen ersten Abschnittsbereich (211a) enthält, der während der Anwendung mit der Mundhöhle des Patienten in Kontakt steht, und einen zweiten Abschnittsbereich (211b) enthält, der vom ersten Abschnittsbereich (211a) abgehoben ist, so dass während der Anwendung eine Lücke zwischen dem zweiten Abschnittsbereich (211b) und der Mundhöhle des Patienten gebildet wird, und wobei der zweite Abschnittsbereich (211b) zwischen dem Befestigungsabschnitt (212) und dem ersten Abschnittsbereich (211a) angeordnet ist.

9. Freier Zahnfleischtransplantatsatz nach Anspruch 1, der ferner eine Nahtumwicklungsanordnung (3) umfasst,
wobei die Nahtumwicklungsanordnung (3) Folgendes umfasst:
eine Heilungsauflage (31), die einen oberen Endabschnitt (311), einen nach unten verjüngten Halsabschnitt (312) und einen Bolzenabschnitt (313) enthält, wobei diese Abschnitte von einem oberen Ende zu einem unteren Ende in dieser Reihenfolge angeordnet sind, wobei der obere Endabschnitt (311) eine freiliegende obere Endfläche und eine Umfangsfläche und einen Kupplungsschlitz (314), der in der freiliegenden oberen Endfläche gebildet ist, aufweist; und
eine Abdeckhaube (32), die ein elastisches Material enthält und einen Abdeckabschnitt (321) enthält, um mit der dem freiliegenden oberen Endabschnitt (311) der Heilungsauflage (31) einen Kontakt zu bewerkstelligen, und einen Greifabschnitt (322) enthält, um einen Umfang der Außenrandfläche des oberen Endabschnitts (311) zu umschließen, wobei der Greifabschnitt (322) eine innere Schlaufengröße aufweist, die kleiner als eine Größe eines Außenprofils der Heilungsauflage (31) ist,
wobei dann, wenn die Abdeckhaube (32) über die Heilungsauflage (31) gesetzt ist, sich der Greifabschnitt (322) und der Abdeckabschnitt (321) der Abdeckhaube (32) elastisch ausdehnen, um die innere Schlaufengröße des Greifabschnitts (322) derart zu vergrößern, dass der Greifabschnitt (322) eine Anschlagsstufe (33) einem Umfang einer Außenrandfläche der Heilungsauflage (31) bereitstellt, um die Heilungsauflage (31) fest zu greifen.

10. Freier Zahnfleischtransplantatsatz nach Anspruch 1, der ferner eine elastische Abdeckfolie (4) umfasst, die ein elastisch dehnbares Durchgangsloch (41) aufweist,
wobei das elastisch dehnbare Durchgangsloch (31) einen Durchmesser aufweist, der kleiner als ein Durchmesser des kratzfreien Abschnitts (14) der kratzfreien TAD-Vorrichtung (1) ist, und
wobei dann, wenn die elastische Abdeckfolie (4) auf der kratzfreien TAD-Vorrichtung (1) angeordnet und von dem kratzfreien Abschnitt (14) zum Halsabschnitt (13) bewegt wird, das elastisch dehnbare Durchgangsloch (41) zunächst von einer ursprünglichen Größe auf eine erweiterte Größe in dem kratzfreien Abschnitt (14) erweitert wird und dann beim Bewegen zum Halsabschnitt (13) von der erweiterten Größe auf die ursprüngliche Größe elastisch zurückgeführt und wiederhergestellt wird.

## Revendications

1. Kit de greffe gingivale indépendant comprenant :
un ensemble couvrant la plaie (2), comprenant un élément de recouvrement protecteur (21) comprenant une section de recouvrement (211) destinée à recouvrir de manière appropriée une plaie locale à l'intérieur de la cavité buccale d'un patient, une première section de positionnement (213) et une seconde section de positionnement (214) étant prévues dans des sections latérales opposées de la section de recouvrement (211),
et une surface inférieure de la section de recouvrement étant une surface irrégulière (215) correspondant à une forme de la cavité buccale du patient, et
un dispositif d'ancrage temporal, TAD (22) comprenant une tête (221) et une tige filetée externe (222), la tige filetée externe (222) s'étendant en cours d'utilisation à travers la seconde section de positionnement (214), la tête (221) comprenant une section d'extrémité de contact qui est pressée contre l'élément de recouvrement protecteur (21), et l'élément de recouvrement protecteur (21) étant maintenu en place par la première section de positionnement (213) et le TAD (22) pour recouvrir la plaie dans la cavité buccale du patient ; et
un TAD non rayant (1) comprenant :
une section de perçage (11) ayant une pointe pointue (111) et une section d'extrémité élargie (112), une surface conique étant est t définie entre la pointe pointue (111) et la section d'extrémité élargie (112) ;
un filetage mâle (12) prévu sur une circonférence de la surface conique de la section de perçage (11) ;
une section de col (13) reliée à la section d'extrémité élargie (112) de la section de perçage (11) et comprenant une tige (131) ayant une surface externe lisse; et
une section non rayante (14) comprenant une section semi-sphérique supérieure (141) et une section semi-sphérique inférieure (142),
la section semi-sphérique supérieure (141) comprenant une section d'extrémité d'engagement (144) destinée à être engagée dans une pièce à main dentaire, la section d'extrémité d'engagement (144) étant pourvue d'une ouverture (145) qui ne comporte pas d'angle vif, et la section semi-sphérique inférieure (142) comprenant une section d'extrémité d'arrêt (146), est la section d'extrémité d'arrêt (146) ayant un diamètre supérieur au diamètre de la tige (131) de la section de col (13) et étant reliée à une zone centrale de la section de col (13), la section d'extrémité d'arrêt (146) de la section semi-sphérique inférieure (142) ayant une section périphérique externe formant une surface d'arrêt circulaire, et la section non rayante (14) ayant un diamètre global supérieur au diamètre de la section d'extrémité d'arrêt.

2. Kit de greffe gingivale indépendant selon la revendication 1, dans lequel l'ouverture (145) ménagée dans la section d'extrémité d'engagement (144) du TAD non rayant (1) comprend une section de coin arrondie (145a) et une section d'alésage (145b) ayant une aire de section transversale inférieure à une aire de section transversale de la section de coin arrondie (145a),
la section de col (13) du TAD anti-rayures (1) comprend en outre une section d'expansion (132) reliée entre la tige (131) de la section de col (13) et la section d'extrémité d'arrêt (146) de la section semi-sphérique inférieure (142), et
la section d'expansion (132) présente une forme d'entonnoir dont le diamètre augmente progressivement vers la section semi-sphérique inférieure (142).

3. Kit de greffe gingivale indépendant selon la revendication 1, dans lequel l'élément de recouvrement protecteur (21) comprend un matériau silicone de qualité médicale et, lorsqu'il est prévu un autre TAD (22) qui s'étend à travers la première section de positionnement (214), les deux TAD (22) maintiennent conjointement, lors de l'utilisation, l'élément de recouvrement protecteur (21) en place au-dessus de la plaie locale dans la cavité buccale du patient, et
les têtes (221) des deux TAD, lors de l'utilisation, pressent le matériau de silicium en pressant des zones du matériau de silicium situées dans une circonférence des TAD (22), de sorte que les têtes des TAD (221) ne soient pas situées plus haut qu'une surface supérieure de l'élément de recouvrement protecteur (21).

4. Kit de greffe gingivale indépendant selon la revendication 3, comprenant en outre une aiguille de positionnement (6) comprenant une section de poignée (61) et une section de dent (62) connectée à une section d'extrémité de la section de poignée (61), la section de dent (62) comprenant un élément de connexion (621) et une pluralité de dents (622) espacées les unes des autres et montées sur l'élément de connexion (621), et
les dents (622) sont capables de pénétrer dans le matériau silicone de l'élément de revêtement protecteur (21) pour former une pluralité de points de positionnement sur l'élément de revêtement protecteur (21).

5. Kit de greffe gingivale indépendant selon la revendication 1, dans lequel l'élément de recouvrement protecteur (21) est formé par impression tridimensionnelle (3D) en utilisant un matériau résineux, la première section de positionnement (213) est un bord en dents de scie correspondant à un profil des dents du patient et, en utilisation, le bord en dents de scie s'étend vers au moins l'un d'une pluralité d'espaces entre les dents.

6. Kit de greffe gingivale indépendant selon la revendication 5, dans lequel la seconde section de positionnement (214) est réalisée sous forme d'un trou étagé comprenant une première ouverture (214a) et une seconde ouverture (214b) ayant un diamètre supérieur à un diamètre de la première ouverture, et
la tête (221) du TAD (22) a une hauteur inférieure à la profondeur de la seconde ouverture (214b) de sorte que, lorsque le TAD (22) s'étend vers le trou étagé, la tête (221) du TAD (22) est complètement logée dans la seconde ouverture (214b).

7. Kit de greffe gingivale indépendant selon la revendication 5, dans lequel l'élément de recouvrement protecteur (21) comprend en outre une section de fixation (212) qui est, lors de l'utilisation, en contact avec une section externe des dents du patient, et la section de fixation (212) est une paroi ondulée s'étendant intégralement vers le haut à partir du bord en dents de scie de la première section de positionnement (213).

8. Kit de greffe gingivale indépendant selon la revendication 7, dans lequel la section de recouvrement (211) de l'élément de recouvrement protecteur (21) comprend une première zone de section (211a) qui, en utilisation, est en contact avec la cavité buccale du patient et une seconde zone de section (211b) relevée depuis la première zone de section (211a), en utilisation, un espace étant formé entre la seconde zone de section (211b) et la cavité buccale du patient, et la seconde zone de section (211b) étant située entre la section de fixation (212) et la première zone de section (211a).

9. Kit de greffe gingivale indépendant selon la revendication 1, comprenant en outre un ensemble d'enroulement de suture (3), l'ensemble d'enroulement de suture (3) comprenant :
une butée de cicatrisation (31) comprenant une section d'extrémité supérieure (311), une section de col effilée vers le bas (312) et une section à boulon (313), disposées séquentiellement d'une extrémité supérieure à une extrémité inférieure, la section d'extrémité supérieure (311) présentant une surface d'extrémité supérieure exposée et une surface périphérique, et une fente de couplage (314) étant formée dans la surface d'extrémité supérieure exposée ; et
un capuchon (32) comprenant un matériau élastique et comprenant une section de recouvrement (321) pour entrer en contact avec la section d'extrémité supérieure exposée (311) de la butée de cicatrisation (31) et une section de préhension (322) pour entourer une circonférence de la surface périphérique de la section d'extrémité supérieure (311), la section de préhension (322) ayant une taille de boucle intérieure inférieure à la taille d'un profil extérieur de la butée de cicatrisation (31),
lorsque le capuchon (32) est ajusté sur la butée de cicatrisation (31), la section de préhension (322) et la section de recouvrement (321) du capuchon (32) se dilatant élastiquement pour augmenter la taille de la boucle intérieure de la section de préhension (322), de sorte que la section de préhension (322) forme un échelon d'arrêt (33) sur une circonférence d'une surface périphérique de la butée de cicatrisation (31) pour saisir fermement la butée de cicatrisation (31).

10. Kit de greffe gingivale indépendant selon la revendication 1, comprenant en outre un film de recouvrement élastique (4) ayant un trou traversant élastiquement dilatable (41),
le trou traversant élastiquement dilatable (31) ayant un diamètre inférieur à un diamètre de la section non rayante (14) du TAD non rayant (1) et,
lorsque le film de recouvrement élastique (4) est placé sur le TAD non rayant (1) et déplacé de la section non rayante (14) à la section de col (13), le trou traversant élastiquement dilatable (41) étant d'abord dilaté d'une taille originale à une taille dilatée dans la section non rayante (14), puis, tout en se déplaçant vers la section de col (13), étant élastiquement ramené de sa taille dilatée à sa taille originale.
